# EUROPÄISCHE PATENTANMELDUNG

(11) **EP 1 350 534 A1**
(43) Veröffentlichungstag der Anmeldung: **08.10.2003**
(21) Anmeldenummer: 03005407.6
(22) Anmeldetag: 13.03.2003
(51) Int. Cl.: A61M 25/02, A61F 13/02

(54) **Pflaster zur Fixierung von Objekten an einem Körper**

(30) Priorität: 20.03.2002 DE 10212393
(71) Anmelder: BSN medical GmbH & Co. KG, 20253 Hamburg (DE)
(72) Erfinder: Mayan, Robert, Dr., 21614 Buxtehude (DE); Goetz, Gabriela, 22395 Hamburg (DE); Rehders, Ingo, 22607 Hamburg (DE); Andrews, Hugh, 25337 Elmshorn (DE); Ramminger, Ralf, 22621 Bargteheide (DE)
(74) Vertreter: UEXKÜLL & STOLBERG

(57) **Zusammenfassung**

Die Erfindung betrifft ein Pflaster zur Fixierung von Objekten, insbesondere von Kanülen, Tuben, Schläuchen, Kathetern und ähnlichem, an einem Körper. Das Pflaster wird am Körper befestigt mittels eines Befestigungsabschnittes (2), der eine zum Aufkleben des Pflasters (1) auf die Haut dienende hautseitige Klebefläche (4) aufweist, wobei der Befestigungsabschnitt (2) auf der hautabgewandten Seite mindestens eine jeweils von einem Rand
ausgehende, hautabgewandte, einen doppelseitig klebenden Bereich des Befestigungsabschnittes (2) definierende Klebefläche (5) und jeweils mindestens einen, einer solchen hautabgewandten Klebefläche (5) zugeordneten, von einem ihrer Ränder ausgehenden Fixierabschnitt (3) aufweist, der in Bezug auf den Befestigungsabschnitt (2) umklappbar und einseitig mit einer Klebefläche (8) versehen ist, so daß diese durch Umklappen des Fixierabschnittes (3) auf die zugehörige hautabgewandte Klebefläche (5) des Befestigungsabschnittes (2), ein auf diese aufgeklebtes Objekt und die an die hautabgewandte Klebefläche (5) des Befestigungsabschnittes (2) angrenzende Körperoberfläche aufgeklebt werden kann.

## Beschreibung

Die vorliegende Erfindung betrifft ein Pflaster zur Fixierung von Objekten, insbesondere von Kanülen, Tuben, Schläuchen, Kathetern und ähnlichem, an einem Körper.

In der Medizin besteht die Notwendigkeit, Kanülen, Tuben, Schläuche, Katheter und ähnliche Objekte schnell und einfach an dem Körper eines Patienten zu befestigen und sicher zu fixieren. Das betrifft beispielsweise Ernährungssonden für enterale Ernährung oder Beatmungsschläuchen, die an der Nase fixiert werden müssen, ableitende Systeme wie Katheter und Drainageschläuche, Ernährungssonden für enterale Ernährung über eine Fistel in der Bauchdecke, suprapubische Katheter, Nierenfisteln zur Nierenpunktion oder Dialyseschläuche sowie verschiedene Zugänge für Medikation, Volumen, Analyse oder Ernährung, etwa zentrale Venenzugänge über die vena subclavia, vena jugulavis oder vena basilica, Meßsonden für die Messung von Herzströmen oder periphere Zugänge, die am Handrücken, der Armbeuge oder am Fußgelenk befestigt werden müssen. In diesen unterschiedlichen Anwendungen müssen die Objekte an verschiedensten Körperregionen mit unterschiedlichst geformten Oberflächen befestigt werden.

Es ist bekannt, diese Fixierungen mit Hilfe eines Pflasters zu bewerkstelligen. Übliche Fixierpflaster sind meist einfache, flächige Klebestreifen, die aus einem Trägermaterial auf Film-, Vlies- oder Gewebebasis bestehen, das einseitig mit einer Haftklebemasse beschichtet ist. Bei der Applikation derartiger Fixierpflaster wird das Objekt zum Beispiel direkt in der gewünschten Lage auf die Körperoberfläche gelegt und das Pflaster anschließend über das Objekt gelegt und mit der Haut verklebt. Diese Prozedur ist von einer Person nur unter großen Schwierigkeiten durchzuführen, da eine Reihe von komplizierten Handhabungen durchzuführen sind, wie das Entfernen von Trennpapieren, die zumeist die Klebefläche oder die Klebeflächen des Fixierpflasters abdecken, bei gleichzeitigem Festhalten des zu befestigenden Objektes in der korrekten Lage. Es besteht die Gefahr, daß das Objekt in einer falschen Position oder unter Zug, unter Druck oder mit einer ungünstigen Abknickung fixiert wird oder daß Klebeflächen des Pflasters aneinander geraten, das Pflaster mit sich selbst verklebt und dadurch unbrauchbar wird. Diese Nachteile treten auch auf, wenn das Pflaster zunächst unter Verklebung mit dem Objekt derart um dieses gewickelt wird, daß zwei freie Pflasterenden verbleiben, die dann auf die Haut aufgeklebt werden können. Aus diesem Grunde ist zumeist die Anwesenheit von zwei Personen erforderlich. Insgesamt ist die Applikation dieser üblichen Fixierpflaster sowohl vom Zeit- als auch vom Personalaufwand aufwendig und daher umständlich und teuer.

Ein Fixierpflaster ist aus EP 0 505 804 B1 bekannt. Dieses Pflaster, das zur Fixierung eines aus der Körperoberfläche austretenden Objektes dient, weist zwei Befestigungsstreifen auf, die schraubenlinienförmig in entgegengesetzter Richtung um das zu befestigende Objekt gewickelt werden. Diese wendelförmige Wicklung der Befestigungsstreifen ist aufwendig und störanfällig. Zudem ist das Pflaster für die Fixierung eines auf der Körperoberfläche liegenden Objektes nicht speziell geeignet.

WO 99/36117 beschreibt ein Fixierpflaster zur Befestigung von Tuben an Mund oder Nase des Patienten. Es besteht aus einem Basisteil und drei, jeweils voneinander separierten, parallel zueinander verlaufenden Befestigungsstreifen, die von dem Basisteil ausgehen. Die beiden äußeren Streifen werden über bzw. unter dem Mund oder den Nasenlöchern auf die Haut geklebt, während der mittlere Streifen wendelförmig um den zu befestigenden Tubus geklebt wird, was kompliziert, aufwendig und mühsam ist.

WO 97/21459 offenbart eine Fixiervorrichtung, die dazu dient, einen im wesentlichen senkrecht aus der Körperoberfläche eines Patienten austretenden Drainageschlauch in unmittelbarer Nähe von der Austrittsstelle knickfrei so umzulenken, daß er parallel zur Körperoberfläche von der Austrittsstelle weggeführt und sicher in dieser Position an der Hautoberfläche fixiert werden kann. Dazu weist das Pflaster eine Pflasterkomponente auf, die auf ihrer Unterseite eine hautseitige, vor der Anwendung mit zwei Trennpapieren abgedeckte Klebefläche hat. In der Pflasterkomponente ist eine Öffnung vorgesehen, durch die der im wesentlichen senkrecht aus dem Körper austretende Drainageschlauch geführt werden kann. Unmittelbar neben der Öffnung ist auf der Oberseite der Pflasterkomponente eine Abstützkomponente befestigt, die eine für den Schlauch angepaßte, von der Öffnung geradlinig wegführende Führungsrinne aufweist. Die Führungsrinne steigt in Richtung zur Öffnung hin an und ist auf ihrer Unterseite mit einem klebenden Material beschichtet, um den Schlauch sicher in der Rinne zu halten. Neben Öffnung und Abstützkomponente ist ein Befestigungsstreifen angeordnet, der in Bezug auf diese umklappbar und einseitig mit einer Klebefläche versehen ist. Der Befestigungsstreifen kann mit seiner Klebefläche über die Abstützkomponente und den von dieser geführten Schlauch sowie den angrenzenden Oberflächenbereich der Pflasterkomponente geklebt werden, um eine sichere Fixierung des Schlauches zu gewährleisten. Dieses Fixierpflaster ist kompliziert aufgebaut und daher teuer und aufwendig in seiner Herstellung. Darüber hinaus ist es speziell zur knickfreien Ableitung eines Drainageschlauches angepaßt, wobei die geeignete Ausrichtung der Führungsrinne bereits vor dem Aufkleben des Pflaster festgelegt werden muß und die Abmessungen der Führungsrinne dem Schlauchdurchmesser annähernd entsprechen müssen.

Es ist Aufgabe der vorliegenden Erfindung, ein Pflaster zur Fixierung von Objekten am Körper so auszugestalten, daß das Pflaster von einer Person einfach, flexibel und schnell angewendet werden kann, um das Objekt sicher in der korrekten Lage zu fixieren, und die genannten Nachteile beseitigt werden.

Zur Lösung dieser Aufgabe dienen die Merkmale von Patentanspruch 1. Vorteilhafte Ausführungsformen des Pflasters sind Gegenstand der zugehörigen Unteransprüche.

Der Grundgedanke der vorliegenden Erfindung besteht darin, daß der Befestigungsabschnitt des Pflasters, mit dem dieses an der Körperoberfläche befestigt wird, nicht nur auf seiner hautseitigen Oberfläche eine Klebefläche aufweist, die zum Aufkleben des Pflasters auf die Haut dient, sondern darüber hinaus zusätzlich eine oder mehrere Klebeflächen auf der hautabgewandten Seite. Diese hautabgewandten Klebeflächen sind auf der hautabgewandten Seite des Befestigungsabschnittes derart angeordnet, daß sie von einem seiner Ränder ausgehen, mit diesem also einen gemeinsamen Randabschnitt haben. Sie definieren zusammen mit den ihnen auf der anderen Seite des Befestigungsabschnittes gegenüberliegenden Bereichen der hautseitigen Klebefläche jeweils einen doppelseitig klebenden Bereich des Befestigungsabschnittes. Auf jede dieser hautabgewandten Klebeflächen kann ein zu fixierendes Objekt aufgeklebt und in einer gewünschten Position gehalten werden, nachdem der Befestigungsabschnitt auf die Haut aufgeklebt und somit das Pflaster in geeigneter Weise an dem Körper befestigt worden ist. Jeder hautabgewandten Klebefläche des Befestigungsabschnittes sind ein oder mehrere Fixierabschnitte zugeordnet. Diese Fixierabschnitte gehen von der hautabgewandten Seite des Befestigungsabschnittes aus und insbesondere von einem Rand der hautabgewandten Klebefläche, so daß ein Rand des Fixierabschnittes mit einem Randabschnitt der Klebefläche zusammenfällt. Die Fixierabschnitte sind relativ zu dem Befestigungsabschnitt derart umklappbar und so dimensioniert, daß sie über die zugehörige hautabgewandte Klebefläche gelegt werden können, dabei über diese und den Befestigungsabschnitt hinausreichen und so einen Teil der angrenzenden Hautoberfläche bedecken können. Zur weiteren Fixierung der auf die zugehörige hautabgewandte Klebefläche aufgebrachten Objekte weisen sie einseitig auf ihrer in der beschriebenen umgeklappten Position hautseitigen Oberfläche eine Klebefläche auf, die somit mit der hautabgewandten Klebefläche des Befestigungsabschnittes, dem Objekt und der an die hautabgewandte Klebefläche angrenzenden Hautoberfläche verklebt werden kann.

Auf diese Weise befinden sich die zu befestigenden Objekte vorteilhaft sowohl von der Ober- als auch von der Unterseite sowie zumindest teilweise auch seitlich mit den Klebeflächen in Kontakt und die Klebeflächen sind jeweils auf beiden Seiten des Objektes mit der Körperoberfläche verklebt. Auf diese Weise wird eine sicherere Fixierung gewährleistet als bei herkömmlichen Fixierpflastern mit nur einer klebenden Oberfläche, die über das Objekt gelegt wird, oder bei Fixierpflastern, die das Objekt umschließende, aber nicht selbst mit der Körperoberfläche verklebte Befestigungsstreifen aufweisen. Somit ist eine erhöhte Sicherheit gegen unbeabsichtigtes Verrutschen des zu fixierenden Objektes gegeben, das etwa bei einem Katheter, einem Tubus oder einer Kanüle weitreichende und schwerwiegende Komplikationen zur Folge haben kann. Die Applikation eines erfindungsgemäßen Fixierpflasters kann einfach und zeitsparend von einer Person durchgeführt werden, indem das Pflaster zunächst mittels der hautseitigen Klebefläche des Befestigungsabschnittes auf der Haut an der gewünschten Position vorfixiert wird. Anschließend kann auf jeder hautabgewandten Klebefläche des Befestigungsabschnittes ein Objekt aufgeklebt und anschließend durch Umklappen des entsprechenden Fixierabschnittes oder der entsprechenden Fixierabschnitte, die dadurch über das Objekt geschlagen werden, endgültig fixiert werden. Die applizierende Person kann das Pflaster somit zuerst beidhändig an der gewünschten Stelle plazieren, dann das zu fixierende Objekt mit beiden Händen positionieren und auf einer hautabgewandten Klebefläche befestigen und schließlich, ebenfalls mit beiden Händen, das vorfixierte Objekt durch Umklappen des mindestens einen Fixierabschnittes sicher befestigen. Das erfindungsgemäße Fixierpflaster erlaubt es also einer einzelnen Person, mit wenigen einfachen Handgriffen eine sichere Fixierung von Objekten am Körper zu bewerkstelligen. Zudem hat das stufenweise Verkleben der einzelnen Klebeflächen den Vorteil, daß diese auch an kompliziert geformten, stark konturierten Körperstellen, wie etwa der Nase, exakt und gezielt positioniert werden können.

In einer bevorzugten Ausführungsform haben der Befestigungsabschnitt und/oder der Fixierabschnitt oder die Fixierabschnitte eine im wesentlichen streifenförmige, rechteckige, quadratische, vieleckige, kreisförmige, kreissegmentförmige, hantelförmige oder ovale Form. Dabei können die einzelnen Fixierabschnitte auch untereinander abweichende Formen aufweisen.

Weiterhin ist es vorteilhaft, wenn die verschiedenen klebenden Flächen des Pflasters vor der Applikation durch Trennpapiere abgedeckt sind. Dazu sind mindestens zwei Trennpapiere vorhanden, wobei die Klebefläche mindestens eines Fixierabschnittes und mindestens eine hautabgewandte Klebefläche des Befestigungsabschnittes durch ein gemeinsames Trennpapier bedeckt sein können. Als Trennpapiere sind nichthaftende flächige Gebilde aus beschichteten oder strukturierten Folien oder Papieren geeignet, insbesondere silikonisierte Papiere. Die Trennpapiere weisen bevorzugt Griffleisten oder Falzungen auf, mit deren Hilfe sie einfacher ergriffen und somit leichter entfernt werden können. Darüber hinaus ist es auch von Vorteil, wenn das die hautzugewandte Klebefläche des Befestigungsabschnittes abdeckende Trennpapier so gefaltet ist, daß es bei auf die Haut gelegtem Befestigungsabschnitt abgezogen werden kann und/oder wenn mindestens eines der eine hautabgewandte Klebefläche des Befestigungsabschnittes abdeckenden Trennpapiere so gefaltet ist, daß es unter einem aufliegenden Objekt weggezogen werden kann. Auf diese Weise müssen die Klebeflächen erst unmittelbar vor der Fixierung des Pflasters und des Objektes freigelegt werden, so daß Pflaster und Objekt zunächst in einfacher Weise korrekt positioniert werden können.

Es ist bevorzugt, daß die Gesamtoberfläche aller doppelseitig klebender Bereiche zwischen 10 und 80% und insbesondere zwischen 20 und 60% der Gesamtoberfläche des Pflasters einnimmt.

In einer bevorzugten Ausgestaltung weist das Fixierpflaster eine oder mehrere flächige Pflasterkomponenten auf, die jeweils aus einem Trägermaterial bestehen, das einseitig mit einer zur Verklebung auf der Haut geeigneten Haftklebermasse beschichtet ist. Das Trägermaterial kann ein Gewebe, ein Vlies, ein Papier, eine Folie oder ein Film oder ein Laminat aus mehreren übereinanderliegenden Schichten eines Gewebes, eines Vlieses, eines Papieres, einer Folie, eines Filmes oder einer Kombination daraus sein.

Es ist weiter vorteilhaft, wenn die hautseitige Klebefläche des Befestigungsabschnittes durch die Klebefläche einer ersten Lage einer derartigen Pflasterkomponente gebildet wird. Jede hautabgewandte Klebefläche des Befestigungsabschnittes kann dann bevorzugt durch jeweils einen Bereich der Klebefläche einer jeweiligen zweite Lage einer solchen Pflasterkomponente gebildet werden, also durch einen Teil der Klebefläche oder aber durch die gesamte Klebefläche. Um einen doppelseitigen Bereiche des Befestigungsabschnittes auszubilden, sind die nichtklebenden Oberflächen der ersten Lage und der jeweiligen zweiten Lage bereichsweise permanent miteinander verbunden. Diese Verbindung kann sowohl außerhalb als auch innerhalb oder nur außerhalb oder nur innerhalb des Bereiches des jeweiligen doppelseitig klebenden Bereiches des Befestigungsabschnittes bestehen. Die Verbindung zwischen der mindestens einen zweiten Lage mit der ersten Lage kann durch einen zwischen ihnen angeordneten Streifen aus einem beidseitig klebenden Material oder durch Verschweißen, Verkleben oder Verschmelzen bewerkstelligt sein.

Dabei ist es möglich, daß die erste Lage und mindestens eine der zweiten Lagen durch separate Pflasterkomponenten gebildet werden. Alternativ kann die erste Lage und mindestens eine der zweiten Lagen integral aus einer einzigen Pflasterkomponente gebildet sein. Diese weist für jede derartige zweite Lage eine erste Falz auf, entlang derer die Pflasterkomponente um im wesentlichen 180° umgeklappt ist, so daß Bereiche der nichtklebenden Rückseite der Pflasterkomponente übereinander liegen.

Jeder Fixierabschnitt und die jeweilige zweite Lage, deren Klebefläche die diesem Fixierabschnitt zugeordnete hautabgewandte Klebefläche bildet, können einstückig durch dieselbe Pflasterkomponente gebildet werden. Es ist dann vorteilhaft, wenn mindestens eine dieser Pflasterkomponenten, die integral eine zweite Lage und einen entsprechenden zugehörigen Fixierabschnitt bilden, eine zweite Falz aufweist, die die Grenze zwischen der jeweiligen hautabgewandten Klebefläche und dem jeweiligen mindestens einen Fixierabschnitt sowie die Umklapprichtung des mindestens einen Fixierabschnittes definiert.

Es ist auch möglich, daß mindestens ein Fixierabschnitt durch eine separate Pflasterkomponente gebildet wird, deren klebende Oberfläche bereichsweise mit einem Bereich der klebenden Oberfläche der zugehörigen zweiten Lage und/oder einem Bereich der nichtklebenden Oberfläche der ersten Lage verbunden ist. Die klebende Oberfläche mindestens einer solchen separaten, einen Fixierabschnitt bildenden Pflasterkomponente und die klebende Oberfläche der zugehörigen zweiten Lage und/oder die nichtklebende Oberfläche der ersten Lage können dann in ihrem Verbindungsbereich mittels eines zwischen ihnen angeordneten Streifens eines beidseitig klebenden Materials aneinander befestigt oder miteinander verschweißt, verklebt oder verschmolzen sein.

In einer weiteren bevorzugten Ausgestaltung ist die Ausrichtung, also die Umklapprichtung, von mindestens einem Fixierabschnitt durch Falzung anpaßbar. Es ist möglich, daß mindestens einer der Fixierabschnitte eine in Bezug auf den Befestigungsabschnitt abweichende Orientierung aufweist.

Die Erfindung wird im folgenden anhand von Ausführungsbeispielen, die in den Zeichnungen dargestellt sind, näher erläutert.
- Figur 1: zeigt eine perspektivische Ansicht eines ersten Fixierpflasters.
- Figur 2: zeigt einen Längsschnitt durch das Fixierpflaster aus Figur 1.
- Figur 3: zeigt einen Längsschnitt durch ein zweites Fixierpflaster.
- Figur 4: zeigt eine Aufsicht von oben auf ein drittes Fixierpflaster.
- Figur 5: zeigt eine Ansicht auf die Stirnseite des Fixierpflasters aus Figur 4 entlang des Pfeils A.

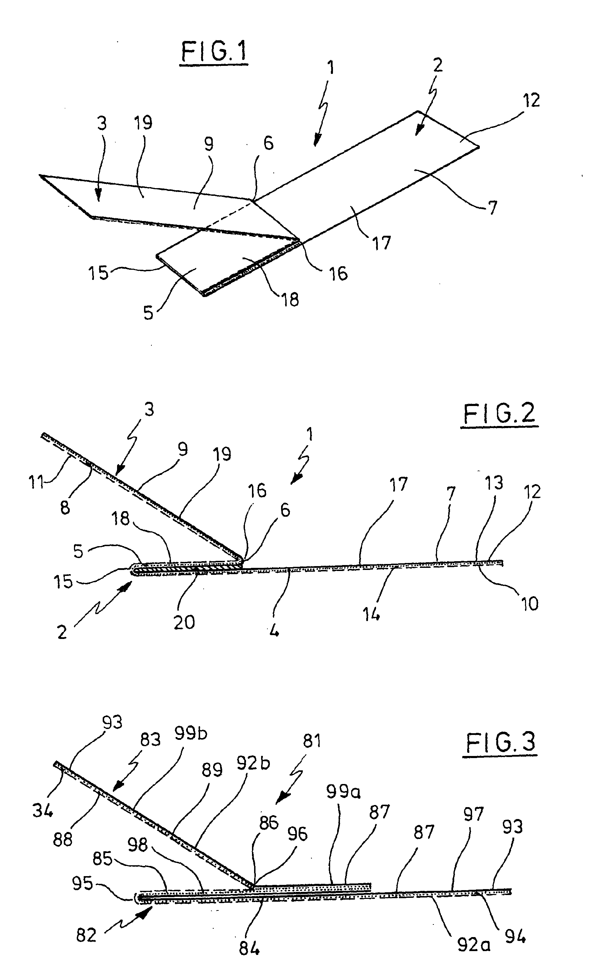

Das in Figur 1 in perspektivischer Ansicht dargestellt Fixierpflaster 1 weist einen streifenförmigen Befestigungsabschnitt 2 und einen streifenförmigen Fixierabschnitt 3 auf. Der Fixierabschnitt 3 ist auf der Oberseite des Befestigungsabschnittes 2 befestigt und in Bezug auf diesen halbkreisförmig um eine zu der Längsachse des Befestigungsabschnittes 2 senkrechte und in dessen Ebene liegende Achse 6 umklappbar. Die Oberfläche des Pflasters 1 ist funktionell in klebende und nicht-klebende Flächen eingeteilt. Die Anordnung dieser Flächen ist insbesondere aus Figur 2 ersichtlich, die einen Längsschnitt durch das in Figur 1 dargestellte Pflaster 1 zeigt. Die Unterseite des Befestigungsabschnittes 2, die bei der Anwendung auf die Hautoberfläche aufgelegt wird, ist vollständig mit einer hautseitigen Klebefläche 4 versehen. Darüber hinaus weist der Befestigungsabschnitt 2 auf seiner gegenüberliegenden, hautabgewandten Seite eine hautabgewandte Klebefläche 5 auf, die den gesamten Oberflächenbereich zwischen einem Rand des Befestigungsabschnittes 2 und der Umklappachse 6 des Fixierabschnittes 3 einnimmt. Dagegen ist die restliche hautabgewandte Oberfläche 7 des Befestigungsabschnittes 2 auf der anderen Seite der Umklappachse 6 nicht-klebend. Der Fixierabschnitt 3 weist einseitig, und zwar auf der der hautabgewandten Klebefläche 5 des Befestigungsabschnittes 2 zugewandten Seite eine weitere Klebefläche 8 auf, während seine gegenüberliegende Fläche 9 nicht klebt. Die Länge des Fixierabschnittes 3 ist so gewählt, daß dieser über den Befestigungsabschnitt 2 hinausragt, wenn der Fixierabschnitt 3 durch Umklappen über dessen hautabgewandte Klebefläche 5 gelegt wird.

Um nun ein Objekt mit Hilfe des Pflaster 1 auf der Hautoberfläche eines Menschen zu befestigen, wird zunächst der Befestigungsabschnitt 2 des Pflasters 1 mit Hilfe seiner hautseitigen Klebefläche 4 auf die Haut aufgeklebt. In einem nächsten Schritt wird das Objekt in der gewünschten Orientierung auf die hautabgewandte Klebefläche 5 des Befestigungsabschnittes 2 aufgeklebt und dadurch vorfixiert. Die endgültige Fixierung erfolgt, indem der Fixierabschnitt 3 um seine Umklappachse 6 über die Klebefläche 5 und das darauf aufgebrachte Objekt geklappt und mit diesen verklebt wird. Aufgrund der Länge des Fixierabschnittes 3 wird dieser dabei auch gleichzeitig mit der an die Klebefläche 5 angrenzenden Hautoberfläche verklebt. Die Klebefläche 8 des Fixierabschnittes 3 ist daher (falls gewünscht auch seitlich) mit dem Objekt, der hautabgewandten Klebefläche 5 auf beiden Seiten des Objektes sowie mit der Haut verklebt und sorgt auf diese Weise zusammen mit der hautabgewandten Klebefläche 5 für eine sichere Fixierung.

Zur leichteren Handhabung sind die Klebeflächen 4, 5, 8 des Pflasters 1 vor der Anwendung durch Trennpapiere 10, 11 abgedeckt (siehe Figur 2), die sich unmittelbar vor der Benutzung der Klebeflächen leicht entfernen lassen. Trennpapier 11 deckt dabei die Klebefläche 8 des Fixierabschnittes 3 und die Klebefläche 5 des Befestigungsabschnittes 2 ab, während Trennpapier 10 die Klebefläche 4 des Befestigungsabschnittes 2 abdeckt. Es ist aber auch genauso möglich, daß die Klebeflächen 5 und 8 jeweils durch separate Trennpapiere abgedeckt werden.

Das Pflaster 1 ist aus einer einzigen Pflasterkomponente 12 gebildet, die aus einem Trägermaterial 13 besteht, das einseitig vollständig von einer Schicht 14 einer Haftklebermasse bedeckt ist. Die Pflasterkomponente 12 weist zwei im wesentlichen senkrecht zu der Längsachse des Pflasters 1 verlaufende, entgegengesetzt gerichtete Falze 15, 16 auf. Durch die Falze 15 und 16 wird die Pflasterkomponente 12 in einen ersten Abschnitt 17 zwischen einem ihrer Enden und der ersten Falz 15, einen zweiten Abschnitt 18 zwischen den beiden Falzen 15 und 16 und einen dritten Abschnitt 19 zwischen der zweiten Falz 16 und dem anderen Ende der Pflasterkomponente 12 eingeteilt. Der erste Abschnitt 17 bildet eine erste, untere Lage des Befestigungsabschnittes 2, und die auf diesem Abschnitt 17 befindliche Klebeschicht 14 bildet dessen hautseitige Klebefläche 4. Die Pflasterkomponente 12 ist entlang der ersten Falz 15 um 180° mit ihrer nicht-klebenden Oberfläche auf sich selbst zurückgeklappt, und die gesamte nicht-klebende Seite des zweiten Abschnittes 18 ist mit Hilfe eines geeignet zugeschnittenen Streifens 20 aus einem doppelseitig klebenden Material permanent auf der nichtklebenden Seite des ersten Abschnittes 17 befestigt. Der zweite Abschnitt 18 bildet eine zweite Lage des Befestigungsabschnittes 2 und die auf ihm befindliche Klebeschicht 14 bildet die hautabgewandte Klebefläche 5 des Befestigungsabschnittes 2. Der Überdeckungsbereich der beiden Abschnitte 17 und 18, also der Bereich der hautabgewandten Klebefläche 5, stellt einen doppelseitig klebenden Bereich des Befestigungsabschnittes 2 dar, während der Rest des Befestigungsabschnittes 2 nur einseitig klebend ist. Der dritte Abschnitt 19 der Pflasterkomponente 12 ist nicht weiter mit den übrigen Abschnitten 17 und 18 verbunden und daher um die zweite Falz 16 umklappbar. Er bildet den Fixierabschnitt 3 mit der durch die zweite Falz 16 vorgegebenen Umklappachse 6.

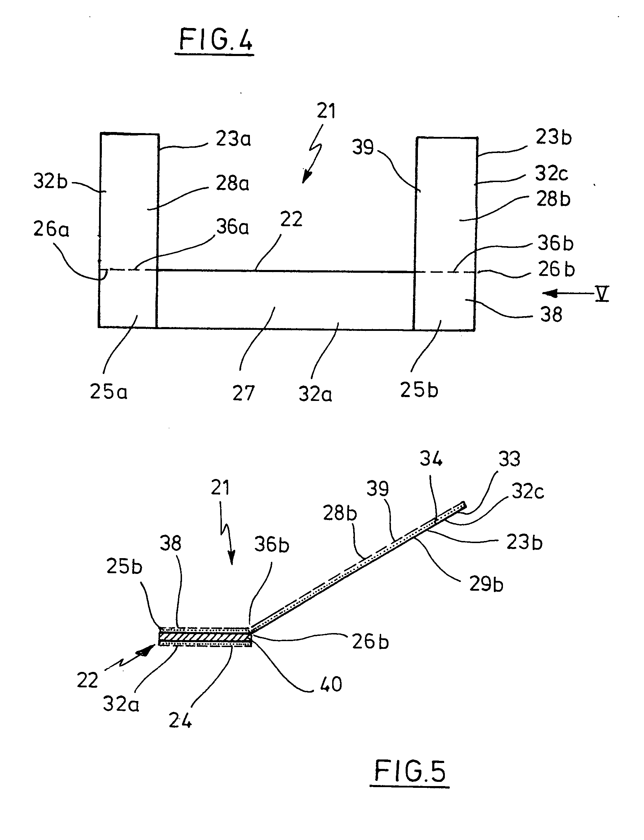

Es ist auch möglich, daß der erste Abschnitt 17 einerseits und der zweite und dritte Abschnitt 18, 19 andererseits von zwei separaten Pflasterkomponenten gebildet werden. Die erste, dem Abschnitt 17 entsprechende Pflasterkomponente stellt dann die erste Lage dar, während die zweite Pflasterkomponente die zweite Lage bildet. Die beiden Lagen sind entsprechend bereichsweise mit ihren nicht-klebenden Oberflächen miteinander verbunden. Ein derartig aufgebautes Pflaster würde dem Pflaster 1 aus Figur 2 gleichen, wenn man die Pflasterkomponente 12 entlang der ersten Falz 15 durchschneiden würde. Ein entsprechend aufgebautes Pflaster wird im Zusammenhang mit den Figuren 4 und 5 beschrieben.

Figur 3 zeigt einen Längsschnitt durch ein zweites erfindungsgemäßes Pflaster 81, dessen perspektivische Ansicht Figur 1 gleichen würde. So weist auch das Pflaster 81 einen streifenförmigen Befestigungsabschnitt 82 und einen auf dessen Oberseite befestigten und in Bezug auf diesen halbkreisförmig um eine zu dessen Längsachse senkrechte und in der Ebene des Befestigungsabschnitts liegende Achse 86 umklappbaren streifenförmigen Fixierabschnitt 83 auf. Genau wie bei Pflaster 1 ist auch der Befestigungsabschnitt 82 des Pflasters 81 an seiner Unterseite, die bei der Anwendung auf die Hautoberfläche aufgelegt wird, vollständig mit einer hautseitigen Klebefläche 84 versehen und weist auf seiner gegenüberliegenden, hautabgewandten Seite eine hautabgewandte Klebefläche 85 auf, die den gesamten Oberflächenbereich zwischen einem Rand des Befestigungsabschnittes 82 und der Umklappachse 86 des Fixierabschnittes 83 einnimmt, während die restliche hautabgewandte Oberfläche 87 des Befestigungsabschnittes 82 auf der anderen Seite der Umklappachse 86 nicht-klebend ist. Der Fixierabschnitt 83 weist einseitig, und zwar auf der der hautabgewandten Klebefläche 85 des Befestigungsabschnittes 82 zugewandten Seite eine weitere Klebefläche 88 auf, während seine gegenüberliegende Fläche 89 nicht klebt. Die Länge des Fixierabschnittes 83 ist so gewählt, daß dieser über den Befestigungsabschnitt 82 hinausragt, wenn der Fixierabschnitt 83 durch Umklappen über dessen hautabgewandte Klebefläche 85 gelegt wird. Aufgrund des dem Pflaster 1 entsprechenden Aufbaus und der entsprechenden funktionellen Anordnung der verschiedenen klebenden und nicht-klebenden Flächen kann ein Objekt in genau der in Bezug auf Pflaster 1 beschriebenen Weise auch mit Hilfe des Pflaster 81 auf der Hautoberfläche eines Menschen befestigt werden.

Anders als das Pflaster 1 ist das Pflaster 81 aber aus zwei separaten Pflasterkomponenten 92a, 92b gebildet, die jeweils aus einem Trägermaterial 93 bestehen, das einseitig vollständig von einer Schicht 94 einer Haftklebermasse bedeckt ist. Die erste Pflasterkomponente 92a weist eine im wesentlichen senkrecht zu der Längsachse des Pflasters 81 verlaufende Falz 95 auf, durch die die Pflasterkomponente 92a in einen ersten Abschnitt 97 zwischen einem ihrer Enden und der Falz 95 und einen zweiten Abschnitt 98 zwischen der Falz 95 und dem anderen Ende der Pflasterkomponente 92a eingeteilt wird. Der erste Abschnitt 97 bildet eine erste, untere Lage des Befestigungsabschnittes 82, und die auf diesem Abschnitt 97 befindliche Klebeschicht 94 bildet dessen hautseitige Klebefläche 84. Die Pflasterkomponente 92a ist entlang der Falz 95 um 180° mit ihrer nicht-klebenden Oberfläche auf sich selbst zurückgeklappt. Ein Bereich der nichtklebende Seite des zweiten Abschnittes 98, der von dem mit dem Ende des zweiten Abschnittes 98 zusammenfallenden Ende der Pflasterkomponente 92a ausgeht, ist unmittelbar permanent durch Verkleben auf der nicht-klebenden Seite des ersten Abschnittes 97 befestigt. Alternativ könnte diese Befestigung auch durch Verschweißen oder Verschmelzen bewirkt werden. Weiterhin ist es auch möglich, daß sich der Bereich, in dem die Abschnitte 97 und 98 aneinander befestigt sind, von dem in Figur 3 dargestellten Bereich abweicht, also in seinen Abmessungen kleiner oder größer ist. So kann der Abschnitt 98 auch mit seiner gesamten nichtklebenden Oberfläche auf dem Abschnitt 97 befestigt sein. Der zweite Abschnitt 98 bildet eine zweite Lage des Befestigungsabschnittes 82 und der Teil der auf ihm befindlichen Klebeschicht 94, der sich zwischen der Falz 95 und der Umklappachse 86 des Fixierabschnittes 83 befindet, bildet die hautabgewandte Klebefläche 85 des Befestigungsabschnittes 82.

Auch die zweite Pflasterkomponente 92b weist eine im wesentlichen senkrecht zu der Längsachse des Pflasters 81 verlaufende Falz 96 auf, durch sie in zwei Abschnitte 99a, 99b eingeteilt wird. Der Abschnitt 99a ist mit seiner gesamten klebenden Oberfläche mit Hilfe eines zusätzlichen Klebemittels permanent auf einen Bereich der klebenden Oberfläche des Abschnittes 98 der Pflasterkomponente 92a aufgeklebt. Auch hier kann die Befestigung des Abschnittes 99a auf dem Abschnitt 98 alternativ mittels Verschweißen oder Verschmelzen bewirkt werden. Dadurch bildet der Abschnitt 99a eine dritte Lage des Befestigungsabschnittes 82 und seine hautabgewandte, nicht-klebende Oberfläche bildet einen Teil der hautabgewandten, nicht-klebenden Oberfläche 87 des Befestigungsabschnittes 82. Der Abschnitt 99b der Pflasterkomponente 92b ist nicht weiter mit dem Abschnitt 99a oder der Pflasterkomponente 92a verbunden und daher um die Falz 96 umklappbar. Er bildet den Fixierabschnitt 83 mit der durch die Falz 96 vorgegebenen Umklappachse 86.

Das Pflaster 81 weist neben den Pflasterkomponenten 92a, 92b keine Komponenten auf, die dem im Pflaster 1 verwendete Streifen 20 aus einem doppelseitig klebenden Material entsprechen. Es bietet daher die Möglichkeit, diese einzusparen, falls dieses aus Kostengründen wünschenswert ist.

Figur 4 zeigt ein weiteres erfindungsgemäßes Pflaster 21 in Aufsicht. Auch dieses Pflaster 21 weist einen Befestigungsabschnitt 22 auf. Anders als Pflaster 1 hat es aber zwei Fixierabschnitte 23a, 23b, die an gegenüberliegenden Enden des Befestigungsabschnittes 22 angeordnet sind. Jeder Fixierabschnitt 23a, 23b ist auf der Oberseite des Befestigungsabschnittes 22 befestigt und in Bezug auf diesen um Umklappachsen 26a, 26b, umklappbar, die mit einem seitlichen Randabschnitt des Befestigungsabschnittes 22 zusammenfallen. Die Unterseite des Befestigungsabschnittes 22, die bei der Anwendung auf die Hautoberfläche aufgelegt wird, ist vollständig mit einer hautseitigen Klebefläche 24 versehen. Auf seiner hautabgewandten Seite weist der Befestigungsabschnitt 22 zwei hautabgewandte Klebeflächen 25a, 25b auf, die jeweils mit einem Fixierabschnitt 23a, 23b funktionell zusammenwirken. Die Klebeflächen 25a, 25b nehmen jeweils den gesamten Oberflächenbereich des Befestigungsabschnittes 22 ein, der beim Umklappen des zugehörigen Fixierabschnittes 23a, 23b überdeckt wird. Dagegen ist die restliche hautabgewandte Oberfläche 27 des Befestigungsabschnittes 22 zwischen den beiden hautabgewandten Klebeflächen 25a, 25b nicht-klebend. Die Fixierabschnitte 23a, 23b weisen einseitig, und zwar auf der den hautabgewandten Klebeflächen 25a, 25b des Befestigungsabschnittes 22 zugewandten Seite jeweils eine weitere Klebefläche 28a, 28b auf, während ihre gegenüberliegenden Flächen 29a, 29b nicht kleben. Die Länge der Fixierabschnitte 23a, 23b ist so gewählt, daß diese über den Befestigungsabschnitt 22 hinausgehen, wenn sie durch Umklappen über die hautabgewandten Klebeflächen 25a, 25b gelegt werden.

Der Aufbau des Pflasters 21 entspricht, abgesehen von der unterschiedlichen Anzahl der hautabgewandten Klebeflächen 25a, 25b und der Fixierabschnitte 23a, 23b sowie deren Ausrichtung in Bezug auf den Befestigungsabschnitt 22, prinzipiell dem Aufbau des Pflasters 1 aus den Figuren 1 und 2. Jedoch wird, wie bereits im Zusammenhang mit diesen Figuren als mögliche Ausgestaltung beschrieben, jede hautabgewandte Klebefläche 25a, 25b des Befestigungsabschnittes 22 und der ihr zugeordnete Fixierabschnitt 23a, 23b durch eine separate zweite Lage einer Pflasterkomponente gebildet. Somit weist das Pflaster 21 drei separate Pflasterkomponenten 32a, 32b, 32c auf, die jeweils aus einem Trägermaterial 33 bestehen, das einseitig vollständig von einer Schicht 34 einer Haftklebermasse bedeckt ist.

Das wird aus Figur 5 deutlich, die eine Ansicht auf die Stirnfläche des Pflasters 21 entlang des Pfeils A in Figur 4 zeigt. Die erste Pflasterkomponente 32a bildet eine erste, untere Lage des Befestigungsabschnittes 22, und die auf dieser Pflasterkomponente 32a befindliche Klebeschicht 24 bildet dessen hautseitige Klebefläche 24. Auf die hautabgewandte, nicht-klebende Oberfläche der Pflasterkomponente 32a ist ein, in Form und Größe den hautabgewandten Klebeflächen 25a, 25b des Befestigungsabschnittes 22 entsprechender Streifen 40 aus einem doppelseitig klebenden Material aufgebracht. Die zweite Pflasterkomponente 32c weist eine im wesentlichen senkrecht zu ihrer Längsachse verlaufende Falz 36b auf, durch die sie in zwei Abschnitte 38, 39 eingeteilt wird. Der Abschnitt 38 entspricht in Form und Größe dem Streifen 40 und ist auf diesen mit seiner nicht-klebenden Oberfläche deckend permanent aufgeklebt. Dadurch bildet der Abschnitt 38 eine zweite Lage des Befestigungsabschnittes 22 und die auf ihm befindliche Klebeschicht 34 bildet die hautabgewandte Klebefläche 25b des Befestigungsabschnittes 22. Der Überdeckungsbereich der beiden Pflasterkomponenten 32a und 32c, also der Bereich der hautabgewandten Klebefläche 25b, stellt einen doppelseitig klebenden Bereich des Befestigungsabschnittes 22 dar, während der Rest des Befestigungsabschnittes 22, mit Ausnahme des Bereiches der zweiten hautabgewandten Klebefläche 25a, nur einseitig klebend ist. Der Abschnitt 39 der Pflasterkomponente 32c ist nicht weiter mit dem Abschnitt 38 oder der Pflasterkomponente 32a verbunden und daher um die Falz 36b umklappbar. Er bildet den Fixierabschnitt 23b mit der durch die Falz 36b vorgegebenen Umklappachse 26b.

Auch bei diesem Pflaster 21 sind die Klebeflächen 24, 25a, 25b, 28a, 28b vor der Anwendung durch Trennpapiere abgedeckt, die sich unmittelbar vor der Benutzung der Klebeflächen leicht entfernen lassen. In diesem Fall deckt aber jeweils ein Trennpapier die Klebeflächen 28a, 28b der Fixierabschnitte 23a, 23b ab, während separate Trennpapiere jeweils eine der Klebeflächen 24 und 25a, 25b des Befestigungsabschnittes 22 abdecken. Es ist aber auch genauso möglich, daß die Klebeflächen 24 und 25a, 25b durch ein einziges Trennpapier abgedeckt werden.

Die Anwendung des Pflasters 21 erfolgt in zu der Anwendung des Pflasters 1 analoger Weise, wobei aber das längliche Objekt im wesentlichen in Richtung der Längsachse des Befestigungsabschnittes 22 verlaufend fixiert wird.

## Patentansprüche

1. Pflaster zur Fixierung von Objekten, insbesondere von Kanülen, Tuben, Schläuchen, Kathetern und ähnlichem, an einem Körper mittels eines Befestigungsabschnittes (2; 22; 42; 82), der eine zum Aufkleben des Pflasters (1; 21; 41; 81) auf die Haut dienende hautseitige Klebefläche (4; 24; 84) aufweist, wobei der Befestigungsabschnitt (2; 22; 42; 82) auf der hautabgewandten Seite mindestens eine jeweils von einem Rand ausgehende, hautabgewandte, einen doppelseitig klebenden Bereich des Befestigungsabschnittes (2; 22; 42; 82) definierende Klebefläche (5; 25a, 25b; 45; 85) und jeweils mindestens einen, einer solchen hautabgewandten Klebefläche (5; 25a, 25b; 45; 85) zugeordneten, von einem ihrer Ränder ausgehenden Fixierabschnitt (3; 23a, 23b; 43; 83) aufweist, der in Bezug auf den Befestigungsabschnitt (2; 22; 42; 82) umklappbar und einseitig mit einer Klebefläche (8; 28a, 28b; 48a, 48b; 88) versehen ist, so daß diese durch Umklappen des Fixierabschnittes (3; 23a, 23b; 43; 83) auf die zugehörige hautabgewandte Klebefläche (5; 25a, 25b; 45; 85) des Befestigungsabschnittes (2; 22; 42; 82), ein auf diese aufgeklebtes Objekt und die an die hautabgewandte Klebefläche (5; 25a, 25b; 45; 85) des Befestigungsabschnittes (2; 22; 42; 82) angrenzende Körperoberfläche aufgeklebt werden kann.

2. Pflaster nach Anspruch 1, bei dem der Befestigungsabschnitt (2; 22; 42; 82) und/oder der mindestens eine Fixierabschnitt (3; 23a, 23b; 43; 83) im wesentlichen streifenförmig, rechteckig, quadratisch, vieleckig, kreisförmig, kreissegmentförmig, hantelförmig oder oval ausgebildet ist.

3. Pflaster nach Anspruch 1 oder 2, bei dem die klebenden Flächen (4, 5, 8; 24, 25a, 25b, 28a, 28b; 45, 48a, 48b) durch mindestens zwei Trennpapiere (10, 11) abgedeckt werden.

4. Pflaster nach Anspruch 3, bei dem die Trennpapiere (10, 11) nichthaftende flächige Gebilde aus beschichteten oder strukturierten Folien oder Papieren sind.

5. Pflaster nach Anspruch 3, bei dem die Trennpapiere (10, 11) silikonisierte Papiere sind.

6. Pflaster nach einem der Ansprüche 3-5, bei dem die hautseitige Klebefläche (4; 24; 84) des Befestigungsabschnittes (2; 22; 42; 82) und mindestens eine hautabgewandte Klebefläche (5; 25a, 25b; 45; 85) durch ein einziges Trennpapier (10, 11) abgedeckt werden.

7. Pflaster nach einem der Ansprüche 3-6, bei dem die Trennpapiere (10, 11) zur verbesserten Entfernbarkeit Griffleisten oder Falzungen aufweisen.

8. Pflaster nach einem der Ansprüche 3-7, bei dem das die hautzugewandte Klebefläche (4; 24; 84) des Befestigungsabschnittes (2; 22; 42; 82) abdeckende Trennpapier (10) so gefaltet ist, daß es bei auf die Haut gelegtem Befestigungsabschnitt (2; 22; 42; 82) abgezogen werden kann.

9. Pflaster nach einem der Ansprüche 3-8, bei dem mindestens eines der eine hautabgewandte Klebefläche (5; 25a, 25b; 45; 85) des Befestigungsabschnittes (2; 22; 42; 82) abdeckenden Trennpapiere (11) so gefaltet ist, daß es unter einem aufliegenden Objekt weggezogen werden kann.

10. Pflaster nach einem der vorhergehenden Ansprüche, bei dem die Oberflächen der doppelseitig klebenden Bereiche 10 - 80% der Gesamtoberfläche des Pflasters (1; 21; 41; 81) einnehmen.

11. Pflaster nach Anspruch 10, bei dem die Oberflächen der doppelseitig klebenden Bereiche 20 - 60% der Gesamtoberfläche des Pflasters (1; 21; 41; 81) einnehmen.

12. Pflaster nach einem der vorhergehenden Ansprüche, das mindestens eine flächige Pflasterkomponente (12; 32a, 32b, 32c; 92a, 92b) aufweist, die aus einem Trägermaterial (13; 33; 93) besteht, das einseitig mit einer zur Verklebung auf der Haut geeigneten Haftklebemasse beschichtet ist.

13. Pflaster nach Anspruch 12, bei dem das Trägermaterial (13; 33; 93) ein Gewebe, ein Vlies, ein Papier, eine Folie oder ein Film ist.

14. Pflaster nach Anspruch 12, bei dem das Trägermaterial (13; 33; 93) ein Laminat aus Schichten eines Gewebes, eines Vlieses, eines Papieres, ein Filmes, einer Folie oder einer Kombination daraus ist.

15. Pflaster nach einem der Ansprüche 12-14, bei dem die hautseitige Klebefläche (4; 24; 84) des Befestigungsabschnittes (2; 22; 42; 82) durch die Klebefläche einer ersten Lage einer solchen Pflasterkomponente (12; 32a; 92a) gebildet wird.

16. Pflaster Anspruch 15, bei dem jede hautabgewandte Klebefläche (5; 25a, 25b; 45; 85) des Befestigungsabschnittes (2; 22; 42; 82) jeweils durch einen Bereich der Klebefläche einer jeweiligen zweiten Lage einer solchen Pflasterkomponente (12; 32b, 32c; 92a) gebildet wird, wobei die nichtklebende Oberfläche der jeweiligen zweiten Lage zur Ausbildung des doppelseitig klebenden Bereiches des Befestigungsabschnittes (2; 22; 42; 82) bereichsweise permanent mit der nichtklebenden Oberfläche der ersten Lage verbunden ist.

17. Pflaster nach Anspruch 16, bei dem jede zweite Lage jeweils im Bereich des jeweiligen doppelseitig klebenden Bereiches des Befestigungsabschnittes (2; 22; 42) mit der ersten Lage verbunden ist.

18. Pflaster nach Anspruch 16, bei dem jede zweite Lage jeweils außerhalb des jeweiligen doppelseitig klebenden Bereiches des Befestigungsabschnittes (82) mit der ersten Lage verbunden ist.

19. Pflaster nach einem der Ansprüche 16-18, bei dem mindestens eine der zweiten Lagen mit der ersten Lage mittels eines zwischen ihnen angeordneten Streifens (20; 40) eines beidseitig klebenden Materials verbunden ist.

20. Pflaster nach einem der Ansprüche 16-18, bei dem mindestens eine der zweiten Lagen und die erste Lage in ihrem Verbindungsbereich miteinander verschweißt, verklebt oder verschmolzen sind.

21. Pflaster nach einem der Ansprüche 16-20, bei dem die erste Lage und mindestens eine der zweiten Lagen durch separate Pflasterkomponenten (32a, 32b, 32c) gebildet werden.

22. Pflaster nach einem der Ansprüche 16-20, bei dem die erste Lage und mindestens eine der zweiten Lagen integral aus einer einzigen Pflasterkomponente (12; 92a) gebildet werden, die für jede dieser zweiten Lagen eine erste Falz (15; 95) aufweist, entlang derer die Pflasterkomponente (12; 92a) um im wesentlichen 180° umgeklappt ist.

23. Pflaster nach einem der Ansprüche 16-22, bei dem jeder Fixierabschnitt (3; 23a, 23b; 43) und die zweite Lage, deren Klebefläche die diesem zugeordnete hautabgewandte Klebefläche (5; 25a, 25b; 45) bildet, einstückig durch dieselbe Pflasterkomponente (12; 32b, 32c) gebildet werden.

24. Pflaster nach Anspruch 23, bei dem mindestens eine Pflasterkomponente (21; 32b, 32c), die einstückig eine zweite Lage und den entsprechenden Fixierabschnitt (3; 23a, 23b; 43) bildet, eine zweite Falz (16; 36a, 36b) aufweist, die die Grenze zwischen der jeweiligen hautabgewandten Klebefläche (5; 25a, 25b; 45) und dem jeweiligen mindestens einen Fixierabschnitt (3; 23a, 23b; 43) sowie die Umklapprichtung des mindestens einen Fixierabschnittes (3; 23a, 23b; 43) definiert.

25. Pflaster nach einem der Ansprüche 16-22, bei dem jeder Fixierabschnitt (83) durch eine separate Pflasterkomponente (92b) gebildet wird, deren klebende Oberfläche bereichsweise mit einem Bereich der klebenden Oberfläche der zugehörigen zweiten Lage und/oder einem Bereich der nichtklebenden Oberfläche der ersten Lage verbunden ist.

26. Pflaster nach Anspruch 25, bei dem die klebende Oberfläche mindestens einer separaten Pflasterkomponente (92b), die einen Fixierabschnitt (83) bildet, und die klebende Oberfläche der zugehörigen zweiten Lage und/oder die nichtklebende Oberfläche der ersten Lage in ihrem Verbindungsbereich mittels eines zwischen ihnen angeordneten Streifens (20; 40) eines beidseitig klebenden Materials verbunden ist.

27. Pflaster nach Anspruch 25, bei dem die klebende Oberfläche mindestens einer separaten Pflasterkomponente (92b), die einen Fixierabschnitt (3; 23a, 23b; 43) bildet, und die klebende Oberfläche der zugehörigen zweiten Lage und/oder die nichtklebende Oberfläche der ersten Lage in ihrem Verbindungsbereich miteinander verschweißt, verklebt oder verschmolzen sind.

28. Pflaster nach einem der vorhergehenden Ansprüche, bei dem mindestens einer der Fixierabschnitte (3; 23a, 23b; 43; 83) eine in Bezug auf den Befestigungsabschnitt (2; 22; 42; 82) abweichende Orientierung aufweist.

29. Pflaster nach einem der vorhergehenden Ansprüche, bei dem die Ausrichtung mindestens eines der Fixierabschnitte (3; 23a, 23b; 43; 83) durch Falzung anpaßbar ist.
